# FASCICULE DE BREVET EUROPEEN

(11) **EP 0 669 935 B1**
(45) Date de publication et mention de la délivrance du brevet: **05.06.1996**
(21) Numéro de dépôt: 94900182.0
(22) Date de dépôt: 10.11.1993
(51) Int. Cl.: C07K 5/075, A23L 1/236

(54) **COMPOSES UTILES COMME AGENTS EDULCORANTS, LEUR PROCEDE DE PREPARATION**
ALS SÜSSTOFFE BRAUCHBARE VERBINDUNGEN UND HIR HERSTELLUNGSVERFAHREN
COMPOUNDS USEFUL AS SWEETENING AGENTS AND PROCESS FOR THEIR PREPARATION

(30) Priorité: 12.11.1992 FR 9213615
(43) Date de publication de la demande: 06.09.1995
(73) Titulaire: NOFRE, Claude, F-69003 Lyon (FR); TINTI, Jean-Marie, F-69680 Chassieu (FR)
(72) Inventeur: NOFRE, Claude, F-69003 Lyon (FR); TINTI, Jean-Marie, F-69680 Chassieu (FR)
(74) Mandataire: Hubert, Philippe
(86) Numéro de dépôt international: FR9301103
(87) Numéro de publication internationale: WO9411391

(56) Documents cités:
- EP-A- 0 107 597
- EP-A- 0 334 236
- EP-A- 0 338 946
- JOURNAL OF ENZYME INHIBITION vol. 5, no. 2 , 1991 pages 133 - 149 A. PATEL ET AL. 'Novel Inhibitors of Enkephalin-Degrading Enzymes III: 4-Carboxymethylamino-4-Oxo-3 (Phenylamino) Butanoic Acids as Enkephalinase Inhibitors'
- JOURNAL OF THE AMERICAN CHEMICAL SOCIETY vol. 91, no. 10 , 7 Mai 1969 , WASHINGTON, DC US pages 2684 - 2691 R.H. MAZUR 'Structure-Taste Relationships of Some Dipeptides' cité dans la demande

## Description

La présente invention a pour objet de nouveaux composés dérivant de l'aspartame, utiles comme agents édulcorants, ainsi que leur procédé de préparation.

Ces nouveaux composés sont particulièrement utiles pour édulcorer des produits variés, en particulier les boissons, les aliments, les confiseries, les pâtisseries, les chewing-gums, les produits d'hygiène et les articles de toilette, ainsi que les produits cosmétiques, pharmaceutiques et vétérinaires.

On sait qu'un agent édulcorant, pour être utilisable à l'échelle industrielle, doit posséder, d'une part, un pouvoir sucrant intense, permettant de limiter le coût d'utilisation, et, d'autre part, une stabilité satisfaisante, c'est-à-dire compatible avec les conditions d'utilisation.

Parmi les agents édulcorants actuellement commercialisés, un dérivé dipeptidique, le *N*-L-α-aspartyl-L-phénylalanine 1-méthyl ester, connu sous le nom d'aspartame, de formule suivante: 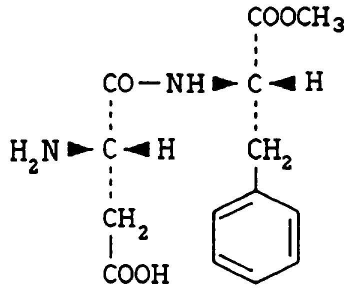 est aujourd'hui le plus utilisé (US 3,492,131). L'un des atouts de ce composé est sa constitution chimique à base de deux amino acides naturels, l'acide L-aspartique et la L-phénylalanine. Le pouvoir sucrant, relativement faible, de ce composé est d'environ 120 à 180 fois celui du saccharose sur une base pondérale. Malgré d'excellentes qualités organoleptiques, ce composé a pour principal inconvénient d'être un produit cher, en raison de son intensité édulcorante relativement basse, et d'avoir une assez faible stabilité dans certaines conditions d'utilisation des édulcorants, notamment en milieu neutre, ce qui limite ses champs d'applications industrielles.

En conséquence, il est apparu nécessaire pour l'industrie alimentaire de pouvoir disposer d'un nouvel agent édulcorant qui présenterait une activité édulcorante élevée, ceci afin de diminuer son coût de revient, et qui serait au moins aussi stable et même plus stable que l'aspartame notamment en milieu neutre. C'est ainsi que de nombreux dipeptides ou analogues dipeptidiques sucrés ont depuis été synthétisés (voir par exemple J.M. Janusz, dans Progress in Sweeteners, Ed. T.H. Grenby, Elsevier, London, 1989, pp. 1-46), mais à ce jour, à part l'aspartame, aucun n'a paru satisfaire aux principales exigences que l'on demande à un édulcorant, à savoir, excellentes qualités organoleptiques, intensité édulcorante suffisamment élevée pour diminuer le coût d'utilisation, stabilité suffisante.

Il a été découvert, de façon tout à fait inattendue, et ceci constitue le fondement de la présente invention, que le pouvoir sucrant de l'aspartame peut être très fortement augmenté en fixant sur l'amine libre de l'aspartame certains radicaux, notamment des radicaux hydrocarbonés convenablement sélectionnés; le pouvoir édulcorant de l'aspartame peut ainsi être multiplié jusqu'à 80 fois, l'intensité édulcorante variant suivant la nature spécifique du radical R.

Des résultats de même nature ont été observés avec les esters éthylique, isopropylique, propylique et tertio-butylique de la *N*-L-α-aspartyl-L-phénylalanine (US 3,492,131) et avec le *N*-L-α-aspartyl-L-tyrosine 1-méthyl ester (US 3,475,403).

Selon un premier aspect, la présente demande vise donc à couvrir les composés de formule : 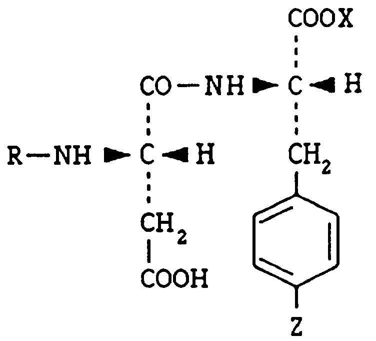 dans laquelle
R est choisi parmi les groupes CH₃(CH₂)₂CH₂, (CH₃)₂CHCH₂, (CH₃)₂CHCH₂CH₂, CH₃CH₂CH(CH₃)CH₂, (CH₃CH₂)₂CHCH₂, (CH₃)₃CCH₂CH₂, cyclohexyle, cycloheptyle, cyclooctyle, cyclopentylméthyle, cyclohexylméthyle, 3-phénylpropyle, 3-méthyl-3-phénylpropyle, 3,3-diméthyl cyclopentyle, 3-méthylcyclohexyle, 3,3,5,5-tétraméthyl cyclohexyle, 2-hydroxycyclohexyle, 3-(4-hydroxy-3-méthoxyphényl)propyle, 3- (4-hydroxy-3-méthoxyphényl)-2-propényle, 3-(4-hydroxy-3-méthoxyphényl)-1-méthylpropyle et 3-(4-hydroxy-3-méthoxyphényl)-1-méthyl-2-propényle;
X est choisi parmi les groupes CH₃, CH₂CH₃, CH(CH₃)₂, CH₂CH₂CH₃ et C(CH₃)₃;
Z représente un atome d'hydrogène ou un groupe OH;
ainsi que les sels physiologiquement acceptables de ces composés.

Des dérivés N-substitués de l'aspartame présentant un pouvoir édulcorant élevé ont déjà été décrits dans l'art antérieur. Ainsi, dans le document EP-0 107 597 (US 4,645,678) sont décrits des composés N-phénylcarbamoyle ou N-phénylthiocarbamoyle de l'aspartame dont le pouvoir sucrant peut atteindre jusqu'à 55 000 fois celui du saccharose. Cependant, il n'existe aucune similitude structurale entre ces groupes *N*-phénylcarbamoyle ou *N*-phénylthiocarbamoyle et les groupes *N*-hydrocarbonés des composés de l'invention.

D'autres dérivés *N*-substitués de l'aspartame ont aussi été décrits (voir, par exemple, J.M. Janusz, citée précédemment), mais il s'agit également de composés ne présentant aucune relation structurale avec les dérivés *N*-hydrocarbonés de l'invention.

En fait, il existait dans l'état de la technique un préjugé défavorable qui a dissuadé jusqu'à présent l'homme de métier de s'orienter vers la recherche de dérivés *N*-hydrocarbonés de l'aspartame à pouvoir édulcorant élevé. C'est ainsi que le seul dérivé *N*-hydrocarboné de l'aspartame décrit dans la littérature, à savoir le *N*-[*N,N*-diméthyl-L-α-aspartyl]-L-phénylalanine 1-méthyl ester, de formule suivante : 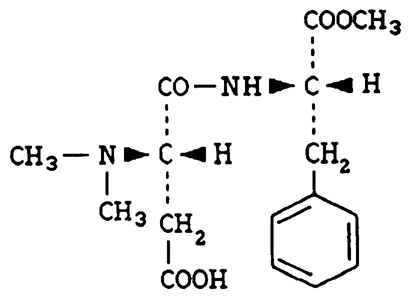 est décrit comme ayant une saveur amère (R.H. Mazur et *al.*, J. Amer. Chem. Soc., 1969, **91**, 2684-2691).

Les recherches conduites par les présents inventeurs ont d'ailleurs permis de constater que les propriétés organoleptiques des dérivés *N*-hydrocarbonés de l'aspartame sont tout à fait imprévisibles et que des groupes hydrocarbonés structuralement très voisins conduisent à des dérivés de l'aspartame qui peuvent être suivant le cas sucrés, sucrés-amers, amers ou insipides. Par ailleurs, dans le document EP-0 338 946 (US 4,935,517), les Demandeurs ont décrits des dérivés *N*-hydrocarbonés des acides L-aspartique (n = 1) ou L-glutamique (n = 2) répondant à la formule générale suivante : 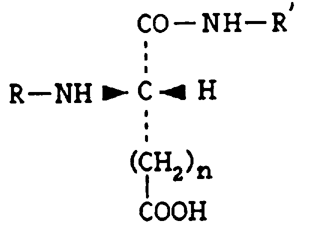 dans laquelle le radical R est un groupe hydrocarboné de cinq à treize atomes de carbone, saturé ou insaturé, acyclique, cyclique ou mixte, dans laquelle le radical R' est un groupe 4-cyanophényle, 2-cyanopyrid-5-yle ou 2-cyanopyrimidin-5-yle, et dans laquelle n est égal à 1 ou 2.

Les composés de l'invention se distinguent de ces composés antérieurs par le fait qu'ils sont spécifiques de l'acide L-aspartique, qu'ils comportent un groupe R' qui ne présente aucune analogie structurale avec ceux définis dans le document EP-0 338 946, et que leur activité dépend de la sélection de groupes *N*-hydrocarbonés bien spécifiques.

Les études de relations structure-activité réalisées par les présents inventeurs ont en effet permis de constater que les groupes *N*-hydrocarbonés les plus efficaces du document antérieur EP-0 338 946 conduisent, par combinaison avec l'aspartame, à des composés amers ou sucrés-amers. Il en est notamment ainsi avec le groupe *n*-heptyle qui, dans le document antérieur, conduit à l'un des composés les plus puissamment sucrés, mais qui, combiné avec l'aspartame, donne un composé avec un très fort arrière-goût amer.

Il a en outre été démontré que la stabilité des composés caractéristiques de l'invention est plus élevée que celle de l'aspartame dans les conditions courantes d'utilisation pour les préparations alimentaires. Ceci est un avantage d'autant plus important que l'une des limites à l'utilisation de l'aspartame dans certaines préparations alimentaires provient de sa stabilité très faible en milieu proche de la neutralité, c'est-à-dire pour des pH voisins de 7, pH qui sont fréquemment rencontrés dans des produits tels que les produits laitiers, les pâtisseries ou autres préparations qui nécessitent une cuisson à haute température, les chewing-gums, les dentifrices.

Une étude de vieillissement accéléré par chauffage prolongé à 70 °C d'une solution aqueuse à pH 7 d'un composé caractéristique de l'invention, à savoir le *N*-[*N*-(3,3-diméthylbutyl)-L-α-aspartyl]-L-phénylalanine 1-méthyl ester dont le pouvoir sucrant est 10 000 fois plus élevé que celui du saccharose, présente une demi-vie d'environ 6 heures, alors que la demi-vie de l'aspartame dans les mêmes conditions n'est que de 10 minutes, ce qui correspond, pour le composé selon l'invention, à une stabilité 36 fois plus élevée que celle de l'aspartame. Des résultats comparables ont été obtenus pour les autres composés caractéristiques de l'invention.

On a également démontré que la stabilité des composés de l'invention est au moins égale, voire améliorée, en milieu acide à pH voisin de 3, pH qui correspond au pH des boissons gazeuses qui constituent l'une des applications majeures des édulcorants.

Du fait de leur pouvoir sucrant élevé, un autre avantage des composés de l'invention, comparativement à l'aspartame, est de permettre, dans leur application aux produits alimentaires, l'utilisation de quantités très faibles d'agent actif. En conséquence, dans les produits alimentaires, la présence souvent débattue de certains constituants de l'aspartame, à savoir la L-phénylalanine et le méthanol, sera très fortement réduite par l'emploi d'un édulcorant de la présente invention. C'est ainsi par exemple qu'il sera possible de remplacer, dans un litre de boisson gazeuse, 550 mg d'aspartame par environ 7 mg du *N*-[*N*-(3,3-diméthylbutyl)-L-α-aspartyl]-L-phénylalanine 1-méthyl ester décrit dans la présente invention, et de diminuer ainsi, jusqu'à 80 fois environ, les quantités de L-phénylalanine et de méthanol susceptibles d'être consommées, tout en maintenant des qualités organoleptiques identiques.

La présente invention permet donc de fournir, pour la première fois, de nouveaux dérivés N-hydrocarbonés de l'aspartame ou de ses analogues qui présentent d'excellentes qualités organoleptiques associées à un pouvoir édulcorant très élevé, jusqu'à 10 000 fois le pouvoir sucrant du saccharose sur une base pondérale, et une stabilité au moins similaire ou plus grande, ce qui, par rapport à l'aspartame, a pour effet d'élargir les possibilités d'utilisation dans les préparations alimentaires.

Une forme de réalisation particulièrement avantageuse de l'invention répond à la formule générale suivante : 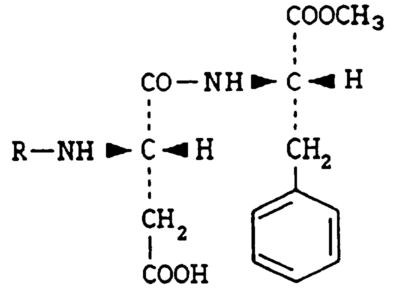 dans laquelle R est tel que défini précédemment.

Un composé répondant à une forme de réalisation particulièrement avantageuse de l'invention est le *N*-[*N*-(3,3-diméthylbutyl)-L-α-aspartyl]-L-phénylalanine 1-méthyl ester (composé 6 du Tableau 1) de formule : 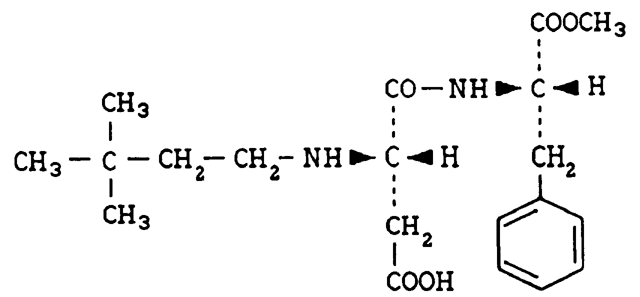 ou le *N*-[*N*-[3-(4-hydroxy-3-méthoxyphényl)propyl]-L-α-aspartyl]-L-phénylalanine 1-méthyl ester (composé 18 du Tableau 1) de formule : 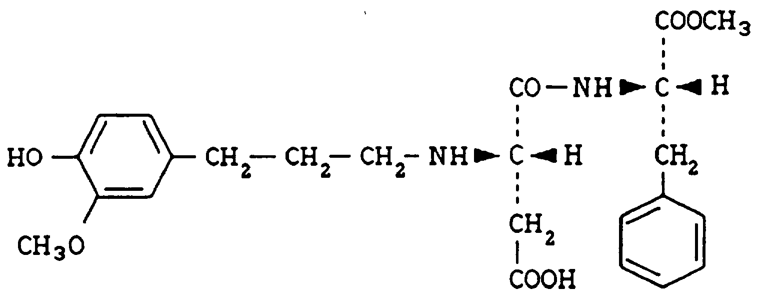 ou encore le *N-[N-*(3-phénylpropyl)-L-α-aspartyl]-L-phénylalanine 1-méthyl ester (composé 12 du Tableau 1) de formule :

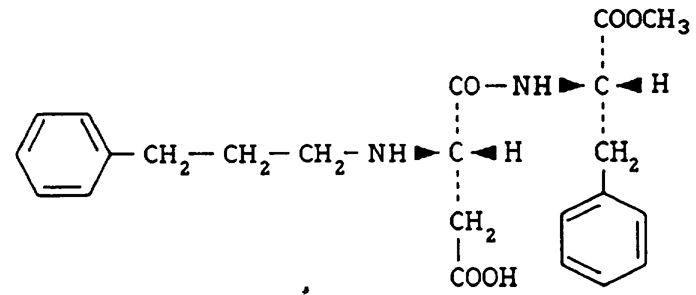

Les composés de l'invention peuvent aussi être salifiés par des acides ou des bases inorganiques ou organiques physiologiquement acceptables, ce qui a pour effet d'accroître leur solubilité. Avantageusement, ces composés sont salifiés sous forme d'hydrochlorure ou de sels de sodium, potassium, ammonium, calcium ou magnésium.

Selon un deuxième aspect, la présente demande vise à couvrir les composés de l'invention en tant qu'agents édulcorants, les compositions édulcorantes incorporant à titre d'agent édulcorant au moins un composé défini tel que précédemment et l'utilisation des composés de l'invention pour édulcorer les produits variés rappelés en introduction.

Les agents édulcorants de la présente invention peuvent être ajoutés à tout produit comestible dans lequel on désire apporter un goût sucré, à condition de les ajouter en proportions suffisantes pour atteindre le niveau de sucrosité désiré. La concentration optimale d'utilisation de l'agent édulcorant dépendra de facteurs divers tels que, par exemple, le pouvoir sucrant de l'agent édulcorant, les conditions de stockage et d'utilisation des produits, les constituants particuliers des produits et le niveau de sucrosité désiré. Toute personne qualifiée peut facilement déterminer la proportion optimale d'agent édulcorant qui doit être employée pour l'obtention d'un produit comestible en réalisant des analyses sensorielles de routine. Les agents édulcorants de la présente invention seront, en général, ajoutés aux produits comestibles dans des proportions allant, suivant le pouvoir édulcorant du composé, de 0,5 mg à 50 mg d'agent édulcorant par kilogramme ou par litre de produit comestible. Les produits concentrés contiendront évidemment des quantités plus élevées d'agent édulcorant, et seront ensuite dilués suivant les intentions finales d'utilisation.

Les agents édulcorants de la présente invention peuvent être ajoutés sous forme pure aux produits à édulcorer, mais, en raison de leur pouvoir sucrant élevé, ils sont généralement mélangés à un support ("carrier") ou à un agent de charge ("bulking agent") approprié.

Avantageusement, les supports ou agents de charge appropriés sont choisis dans le groupe constitué par le polydextrose, l'amidon, les maltodextrines, la cellulose, la méthylcellulose, la carboxyméthylcellulose et autres dérivés de la cellulose, l'alginate de sodium, les pectines, les gommes, le lactose, le maltose, le glucose, la leucine, le glycérol, le mannitol, le sorbitol, le bicarbonate de sodium, les acides phosphorique, citrique, tartrique, fumarique, benzoïque, sorbique, propionique, et leurs sels de sodium, potassium et calcium, et leurs équivalents.

Les agents édulcorants conformes à l'invention peuvent, dans un produit comestible, être employés seuls, comme unique agent édulcorant, ou en combinaison avec d'autres agents édulcorants tels que le saccharose, le sirop de maïs, le fructose, les dérivés ou analogues dipeptidiques sucrés (aspartame, alitame), la néohespéridine dihydrochalcone, l'isomaltulose hydrogéné, le stévioside, les sucres L, la glycyrrhizine, le xylitol, le sorbitol, le mannitol, l'acésulfame, la saccharine et ses sels de sodium, potassium, ammonium et calcium, l'acide cyclamique et ses sels de sodium, potassium et calcium, le sucralose, la monelline, la thaumatine, et leurs équivalents.

Les composés de la présente invention peuvent être préparés par des méthodes variées déjà décrites dans la littérature. Ainsi, selon un troisième aspect, la présente demande vise à couvrir l'une des méthodes préférées qui consiste à condenser un composé de formule : 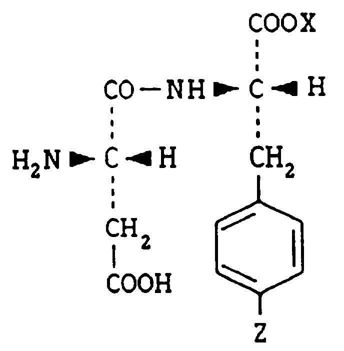 avec un composé aldéhydique ou cétonique précurseur du groupe R. L'imine intermédiaire formée par condensation est alors réduite *in situ* par un agent réducteur sélectif, comme par exemple le cyanoborohydrure de sodium, ce qui conduit directement aux composés de l'invention (méthode de *N*-monoalkylation réductrice décrite par Ohfune *et al.,* Chemistry Letters, 1984, 441-444).

L'obtention, par exemple, d'un composé de l'invention dans lequel R est le radical (CH₃)₃CCH₂CH₂ est effectuée à partir d'un précurseur aldéhydique commercial, le 3,3-diméthylbutyraldéhyde de formule (CH₃)₃CCH₂CHO.

Il est à noter que la préparation des composés de l'invention est directement réalisée à partir de l'aspartame ou de ses analogues. En ce qui concerne les dérivés de l'aspartame, ceci constitue un avantage particulièrement intéressant du fait que l'aspartame est un produit commercial dont la synthèse est aujourd'hui parfaitement maîtrisée.

La purification des composés de l'invention, sous leur forme acide ou salifiée, est réalisée selon les techniques standards telles que la recristallisation ou la chromatographie. Leur structure et leur pureté ont été contrôlées par les techniques classiques (chromatographie sur couche mince, chromatographie liquide haute performance, spectrométrie infrarouge, résonance magnétique nucléaire, analyse élémentaire).

La manière dont l'invention peut être réalisée et les avantages qui en découlent ressortiront mieux des exemples de réalisation qui suivent.

Dans ces exemples, le pouvoir édulcorant des composés décrits a été évalué par un groupe de huit personnes expérimentées. Pour cela, les composés, en solution aqueuse à des concentrations variables, sont comparés, sur le plan gustatif, à une solution témoin de saccharose à 2 %, à 5 % ou à 10 %. Le pouvoir édulcorant du composé, testé par rapport au saccharose, correspond alors au rapport pondéral qui existe entre le composé et le saccharose à égale intensité édulcorante, c'est-à-dire quand les saveurs sucrées de la solution du composé testé et de la solution témoin de saccharose sont considérées, par une majorité de personnes, avoir la même intensité édulcorante.

La stabilité des composés de l'invention et de l'aspartame a été mesurée en dosant, par chromatographie liquide haute performance (HPLC), la quantité de produit restant après un vieillissement accéléré en milieu acide (tampon phosphate à pH 3) ou en milieu neutre (tampon phosphate à pH 7) et à la température de 70 °C. La stabilité du composé ainsi testé est évaluée par sa demi-vie (temps correspondant à 50 % de dégradation).

A titre d'exemple de préparation, la synthèse du *N*-[*N*-(3,3-diméthylbutyl)-L-α-aspartyl]-L-phénylalanine 1-méthyl ester (exemple N° 6 du Tableau 1), de formule : 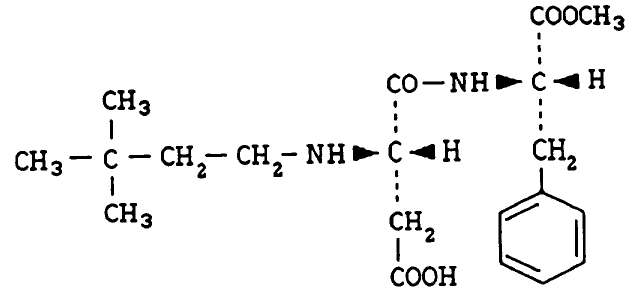 est réalisée de la façon suivante.

Quatre grammes (39,8 mmol) de 3,3-diméthylbutyraldéhyde d'origine commerciale sont ajoutés à un mélange, dans 50 cm³ de méthanol, de 10,6 g (36,2 mmol) d'aspartame et de 1,6 g (25,3 mmol) de cyanoborohydrure de sodium. La solution est agitée durant 24 heures à température ambiante puis est concentrée à sec sous vide. Le résidu est alors repris dans une solution aqueuse d'acide chlorhydrique 1 N jusqu'à ce que le pH soit voisin de la neutralité. Le précipité gommeux formé est séparé par filtration, séché sous vide avant d'être recristallisé dans un mélange éthanol-eau (1-1) ou dans l'acétonitrile. On obtient alors 9 g (rendement 62 %) de *N*-[*N*-(3,3-diméthylbutyl)-L-α-aspartyl]-L-phénylalanine 1-méthyl ester.

Le pouvoir édulcorant de ce composé correspond approximativement, sur une base pondérale, à 10 000 fois celui du saccharose par comparaison avec une solution de saccharose à 2 %, 5 % et 10 %.

Par comparaison avec l'aspartame, une solution aqueuse de 7 mg/L du présent composé est équivalente en intensité édulcorante à une solution de 550 mg/L d'aspartame, ce qui correspond à un pouvoir sucrant environ 80 fois plus élevé que celui de l'aspartame.

A titre d'exemples, le pouvoir sucrant d'autres composés selon l'invention, obtenus à partir de l'aspartame suivant un protocole expérimental similaire à celui décrit ci-dessus et que l'homme de l'art retrouvera facilement, est donné dans le Tableau 1. Le pouvoir sucrant a été évalué par rapport à une solution de saccharose à 2 %.

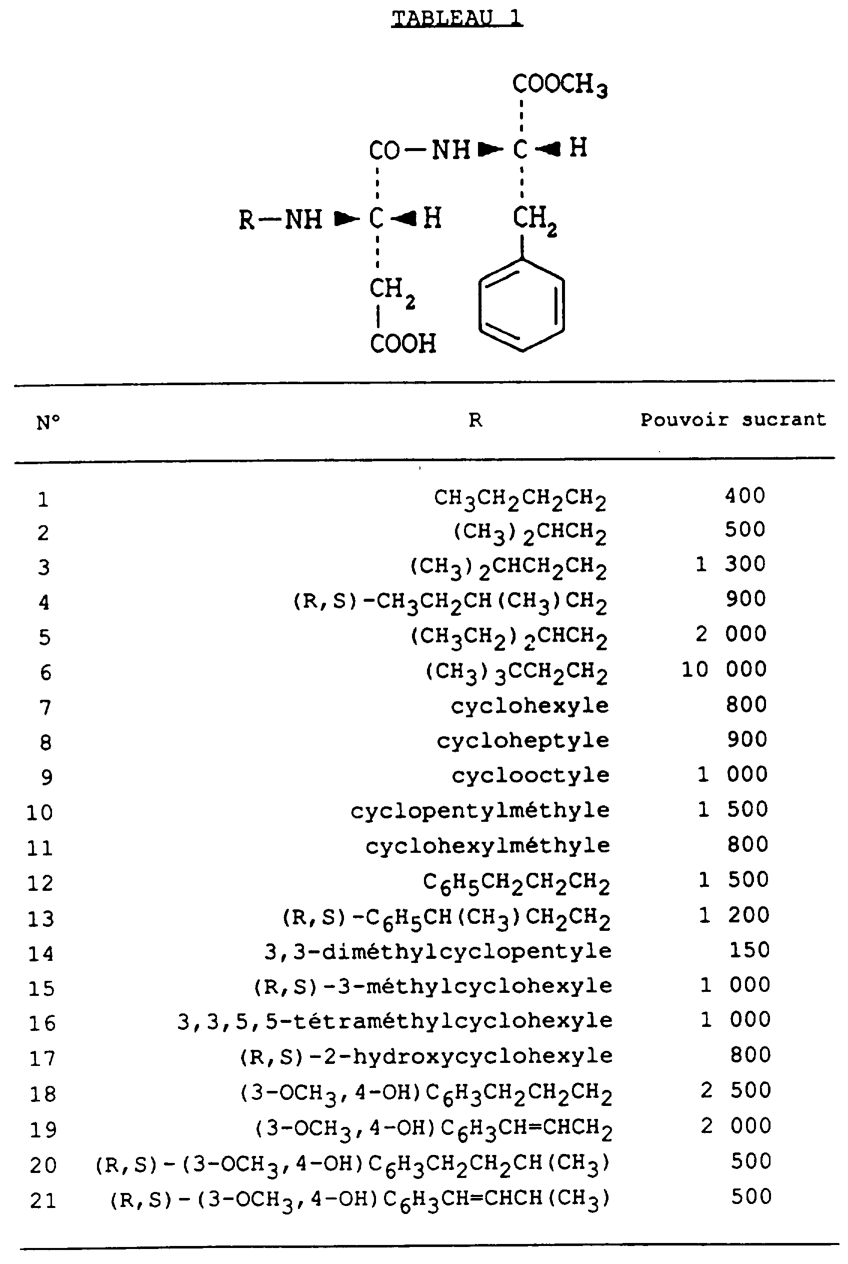

A titre d'exemples additionnels répondant à la formule générale, le *N*-[*N*-(3,3-diméthylbutyl)-L-α-aspartyl]-L-phénylalanine 1-éthyl ester a un pouvoir sucrant de 2 000 fois celui du saccharose, et le *N*-[*N*-(3,3-diméthylbutyl)-L-α-aspartyl]-L-tyrosine 1-méthyl ester a un pouvoir sucrant de 4 000 fois celui du saccharose (par comparaison avec une solution de saccharose à 2 %).

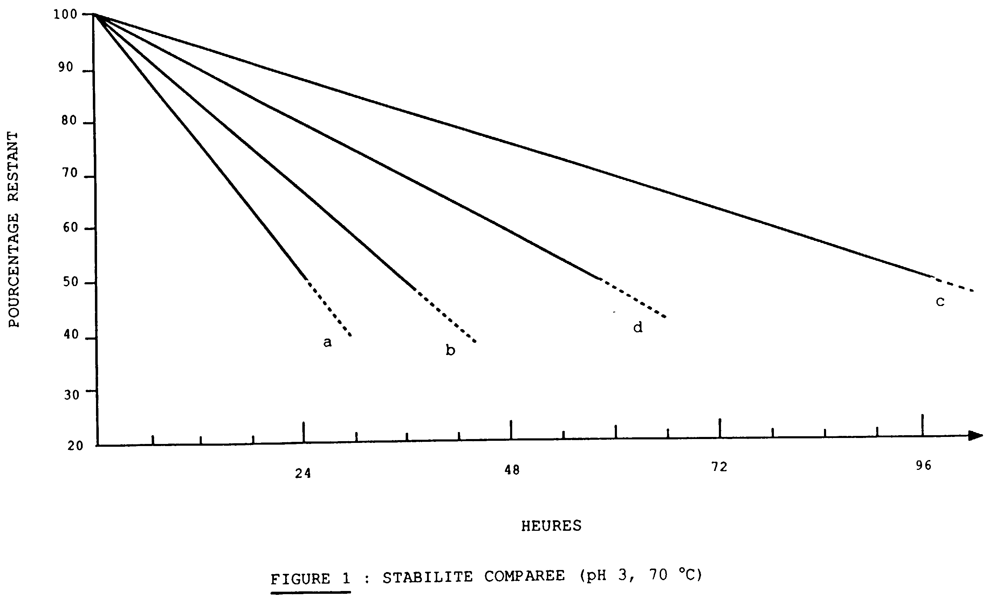

Dans la figure 1 annexée, est donné un diagramme comparatif des courbes de stabilité par rapport à l'aspartame (courbe a), de quelques composés caractéristiques de l'invention, en prenant comme exemples les composés 2, 5 et 6 du Tableau 1 (courbes b, c et d respectivement), ces courbes ayant été obtenues au cours d'un vieillissement accéléré, par chauffage à 70 °C de leurs solutions, à la concentration de 1 g/L, en milieu acide de pH 3. Dans ces conditions expérimentales, la demi-vie de l'aspartame est d'environ 24 heures, alors que les demi-vies des composés de l'invention sont d'environ 35 heures pour le composé 2, 96 heures pour le composé 5 et 55 heures pour le composé 6, ce qui correspond à des stabilités qui sont jusqu'à 4 fois plus élevées que celle de l'aspartame.

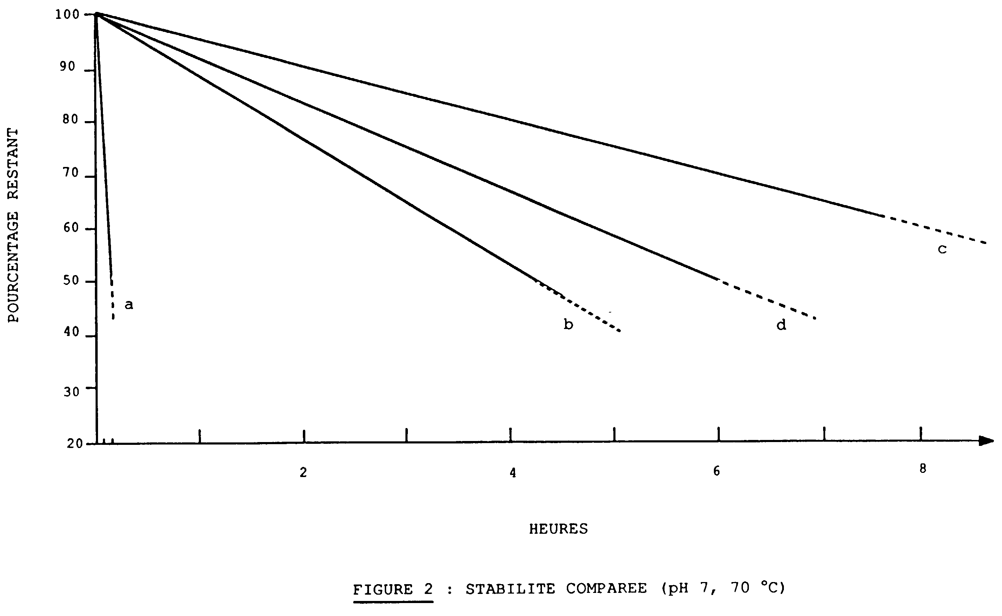

Dans la figure 2 annexée, est donné un diagramme comparatif des courbes de stabilité, par rapport à l'aspartame (courbe a), des composés 2, 5 et 6 du Tableau 1 (courbes b, c et d respectivement), ces courbes ayant été obtenues au cours d'un vieillissement accéléré par chauffage à 70 °C de leurs solutions, à la concentration de 1 g/L, en milieu neutre de pH 7. Dans ces conditions expérimentales, l'aspartame est très peu stable (demi-vie de 10 minutes), alors que les composés de l'invention présentent des demi-vies de 4 h 15 min pour le composé 2, de 10 heures pour le composé 5 et de 6 heures pour le composé 6, ce qui correspond à des stabilités qui sont jusqu'à 60 fois plus élevées que celle de l'aspartame.

## Revendications

1. Composés de formule générale : 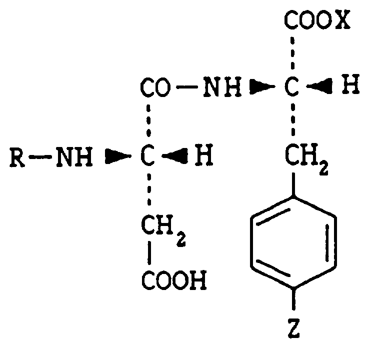 dans laquelle
R est choisi parmi les groupes CH₃(CH₂)₂CH₂, (CH₃)₂CHCH₂, (CH₃)₂CHCH₂CH₂, CH₃CH₂CH(CH₃)CH₂, (CH₃CH₂)₂CHCH₂, (CH₃)₃CCH₂CH₂, cyclohexyle, cycloheptyle, cyclooctyle, cyclopentylméthyle, cyclohexylméthyle, 3-phénylpropyle, 3-méthyl-3-phénylpropyle, 3,3-diméthyl cyclopentyle, 3-méthylcyclohexyle, 3,3,5,5-tétraméthyl cyclohexyle, 2-hydroxycyclohexyle, 3-(4-hydroxy-3-méthoxyphényl)propyle, 3-(4-hydroxy-3-méthoxyphényl)-2-propényle, 3- (4-hydroxy-3-méthoxyphényl)-1-méthylpropyle et 3-(4-hydroxy-3-méthoxyphényl)-1-méthyl-2-propényle;
X est choisi parmi les groupes CH₃, CH₂CH₃, CH(CH₃)₂, CH₂CH₂CH₃ et C(CH₃)₃;
Z représente un atome d'hydrogène ou un groupe OH;
et les sels physiologiquement acceptables de ces composés.

2. Composés selon la revendication 1 caractérisés en ce qu'ils répondent à la formule générale suivante : 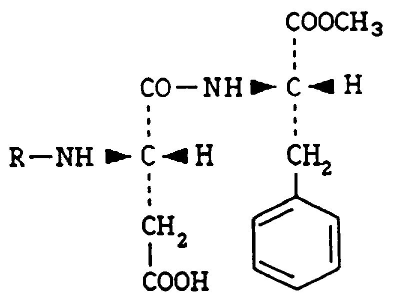 dans laquelle R est tel que défini dans la revendication 1.

3. Composé selon la revendication 2 caractérisé en ce qu'il s'agit du *N*-[*N*-(3,3-diméthylbutyl)-L-α-aspartyl]-L-phénylalanine 1-méthyl ester de formule :

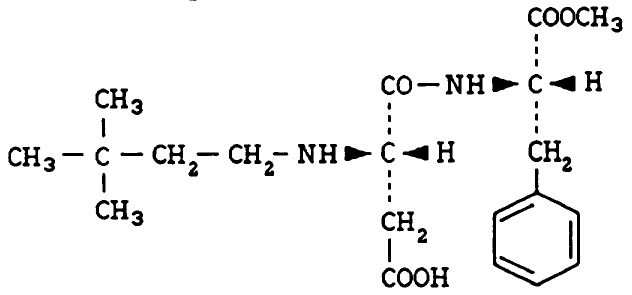

4. Composé selon la revendication 2 caractérisé en ce qu'il s'agit du *N*-[*N*-[3-(4-hydroxy-3-méthoxyphényl) propyl]-L-α-aspartyl]-L-phénylalanine 1-méthyl ester de formule :

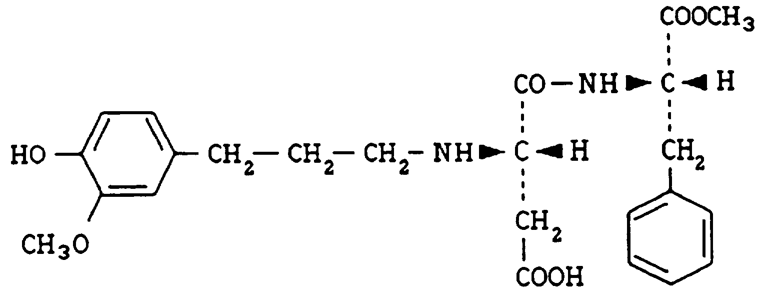

5. Composé selon la revendication 2 caractérisé en ce qu'il s'agit du *N*-[*N*-(3-phénylpropyl)-L-α-aspartyl]-L-phénylalanine 1-méthyl ester de formule :

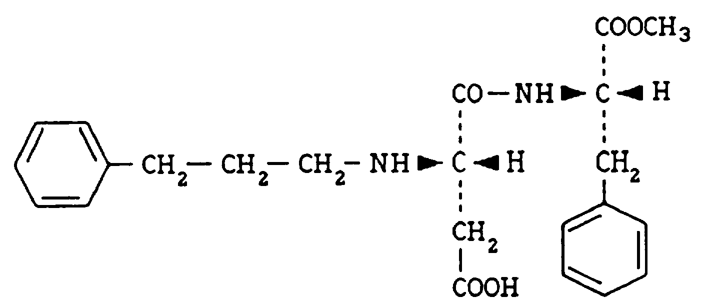

6. Composition édulcorante caractérisée en ce qu'elle comprend à titre d'agent édulcorant au moins un composé selon la revendication 1.

7. Application des composés selon la revendication 1 comme agents édulcorants.

8. Procédé de préparation d'un composé selon la revendication 1 caractérisé en ce qu'il consiste à condenser un composé de formule : 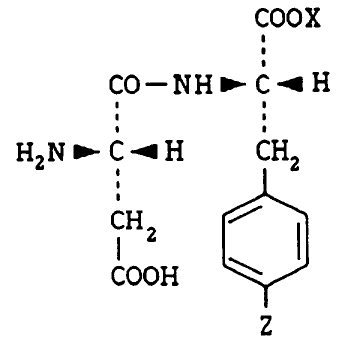 dans laquelle X et Z sont tels que définis dans la revendication 1, avec un précurseur aldéhydique ou cétonique correspondant au groupe R du composé à préparer, puis à réduire *in situ* l'imine résultante par le cyanoborohydrure de sodium, R étant tel que défini dans la revendication 1.

## Patentansprüche

1. Verbindungen der allgemeinen Formel: 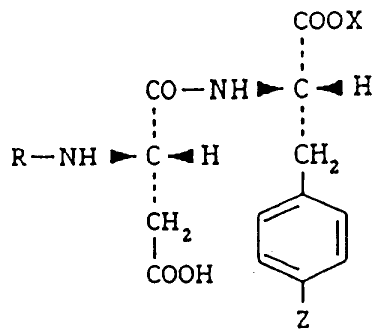
worin R aus den Gruppen CH₃(CH₂)₂CH₂, (CH₃)₂CHCH₂, (CH₃)₂CHCH₂CH₂, CH₃CH₂CH(CH₃)CH₂, (CH₃CH₂)₂CHCH₂, (CH₃)₃CCH₂CH₂, Cyclohexyl, Cycloheptyl, Cyclooctyl, Cyclopentylmethyl, Cyclohexylmethyl, 3-Phenylpropyl, 3-Methyl-3-phenylpropyl, 3,3-Dimethylcyclopentyl, 3-Methylcyclohexyl, 3,3,5,5-Tetramethylcyclohexyl, 2-Hydroxycyclohexyl, 3-(4-Hydroxy-3-methoxyphenyl)propyl, 3-(4-Hydroxy-3-methoxyphenyl)-2-propenyl, 3-(4-Hydroxy-3-methoxyphenyl)-1-methylpropyl und 3-(4-Hydroxy-3-methoxyphenyl)-1-methyl-2-propenyl ausgewählt ist;
X aus den Gruppen CH₃, CH₂CH₃, CH(CH₃)₂, CH₂CH₂CH₃ und C(CH₃)₃ ausgewählt ist;
Z ein Wasserstoffatom oder eine OH-Gruppe darstellt;
und die physiologisch annehmbaren Salze dieser Verbindungen.

2. Verbindungen nach Anspruch 1, dadurch gekennzeichnet, daß sie der folgenden allgemeinen Formel entsprechen: 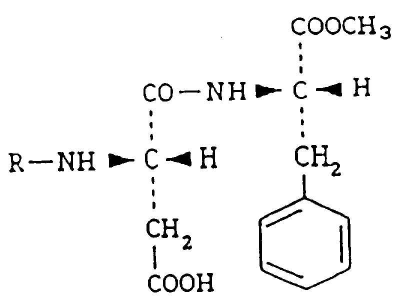 worin R wie in Anspruch 1 definiert ist.

3. Verbindung nach Anspruch 2, dadurch gekennzeichnet, daß es sich um den N-[N-(3,3-Dimethylbutyl)-L-α-aspartyl]-L-phenylalanin-l-methylester der Formel handelt:

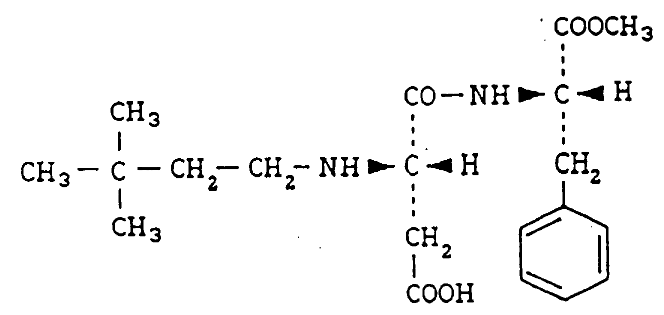

4. Verbindung nach Anspruch 2, dadurch gekennzeichnet, daß es sich um N-[N-[3-(4-Hydroxy-3-methoxyphenyl)propyl]-L-α-aspartyl]-L-phenylalanin-1-methylester der Formel handelt:

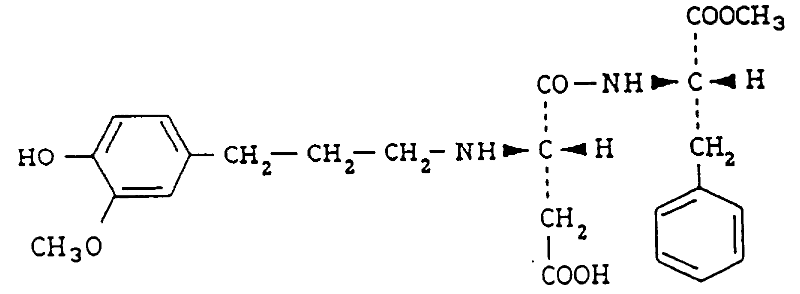

5. Verbindung nach Anspruch 2, dadurch gekennzeichnet, daß es sich um N-[N-(3-Phenylpropyl)-L-α-aspartyl]-L-phenylalanin-1-methylester der Formel handelt:

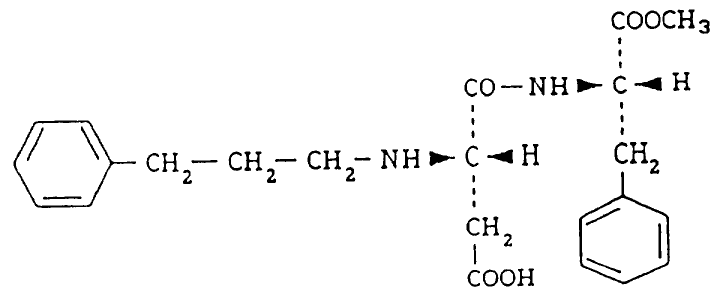

6. Süßstoffzusammensetzung, dadurch gekennzeichnet, daß sie als Süßstoff wenigstens eine Verbindung nach Anspruch 1 enthält.

7. Verwendung von Verbindungen nach Anspruch 1 als Süßstoff.

8. Verfahren zur Herstellung einer Verbindung nach Anspruch 1, dadurch gekennzeichnet, daß es in der Kondensation einer Verbindung der Formel 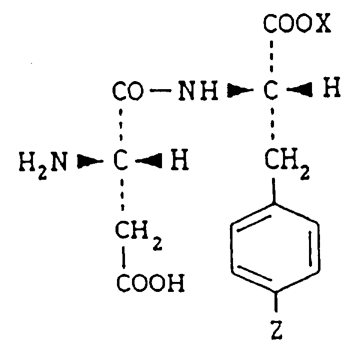 worin X und Z so wie in Anspruch 1 definiert sind, mit einem Vorläuferaldehyd oder -keton entsprechend der Gruppe R der herzustellenden Verbindung besteht, danach in der Reduktion in situ des resultierenden Imins mit Natriumcyanohydrid, wobei R wie in Anspruch 1 definiert ist.

## Claims

1. Compound of the general formula 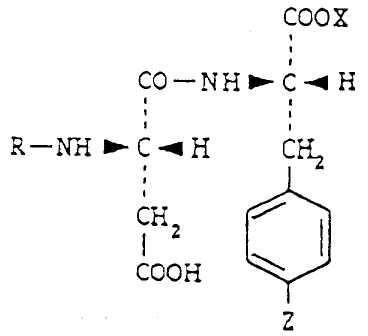
in which R is selected from CH₃(CH₂)₂CH₂, (CH₃)₂CHCH₂, (CH₃)₂CHCH₂CH₂, CH₃CH₂CH(CH₃)CH₂, (CH₃CH₂)₂CHCH₂, (CH₃)₃CCH₂CH₂, cyclohexyl, cycloheptyl, cyclooctyl, cyclopentylmethyl, cyclohexylmethyl, 3-phenylpropyl, 3-methyl-3-phenylpropyl, 3,3-dimethylcyclopentyl, 3-methylcyclohexyl, 3,3,5,5-tetramethylcyclohexyl, 2-hydroxycyclohexyl, 3- (4-hydroxy-3-methoxyphenyl)propyl, 3-(4-hydroxy-3-methoxyphenyl)-2-propenyl, 3-(4-hydroxy-3-methoxyphenyl)-1-methylpropyl and 3-(4-hydroxy-3-methoxyphenyl)-1-methyl-2-propenyl groups;
X is selected from the CH3, CH2CH3, CH(CH3)2, CH2CH2CH3 and C(CH3)3 groups;
Z represents a hydrogen atom or an OH group;
as well as the physiologically acceptable salts of these compounds.

2. Compounds according to claim 1, characterized in that they have the following general formula: 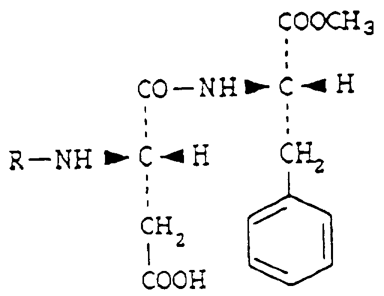 in which R is as defined in claim 1.

3. Compound according to claim 2, characterized in that it is *N-[N*-(3,3-dimethylbutyl)-L-α-aspartyl]-L-phenylalanine 1-methyl ester of the formula

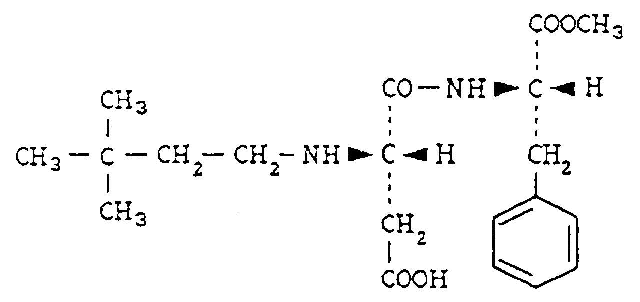

4. Compound according to claim 2, characterized in that it is *N*-[*N*-[3-(4-hydroxy-3-methoxyphenyl)propyl)-L-α-aspartyl]-L-phenylalanine 1-methyl ester of the formula

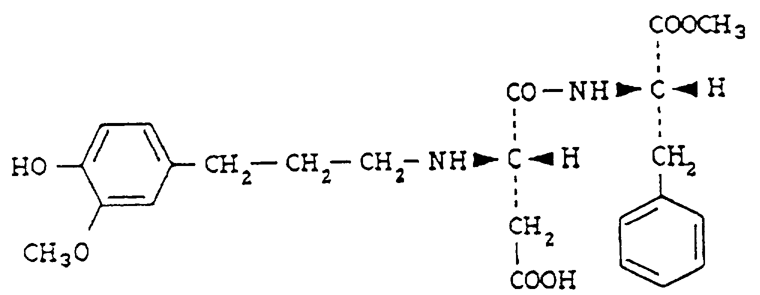

5. Compound according to claim 2, characterized in that it is *N*-[*N*-(3-phenylpropyl)-L-α-aspartyl]-L-phenylalanine 1-methyl ester of the formula

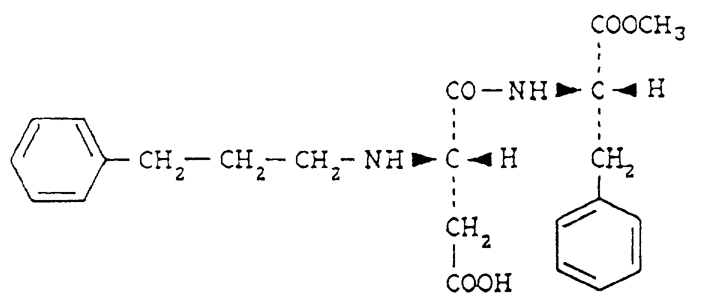

6. Sweetening composition characterized in that it comprises at least one compound according to claim 1 as the sweetening agent.

7. Application of the compounds according to claim 1 as sweetening agents.

8. Method of preparing a compound according to claim 1, characterized in that it consists in condensing a compound of the formula 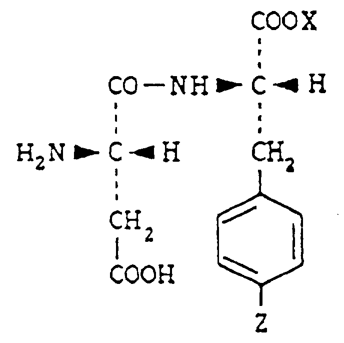 in which X and Z are as defined in claim 1, with an aldehyde or ketone precursor corresponding to the group R of the compound to be prepared, and then reducing the resulting imine *in situ* with sodium cyanoborohydride, R being as defined in claim 1.
